# EUROPEAN PATENT APPLICATION

(11) **EP 3 045 915 A1**
(43) Date of publication of application: **20.07.2016**
(21) Application number: 14844963.0
(22) Date of filing: 10.09.2014
(51) Int. Cl.: G01N 33/569, G01N 33/543, C07K 16/10

(54) **METHOD FOR MEASURING TYPE A INFLUENZA VIRUS**

(30) Priority: 10.09.2013 JP 2013187141
(71) Applicant: Denka Seiken Co., Ltd., Chuo-ku Tokyo 103-8338 (JP)
(72) Inventor: INANO, Koichi, Gosen-shi Niigata 959-1834 (JP); MIYAZAWA, Takashi, Gosen-shi Niigata 959-1834 (JP); ISHIKAWA, Osamu, Gosen-shi Niigata 959-1834 (JP)
(74) Representative: Kremer, Simon Mark
(86) International application number: PCT/JP2014/073949
(87) International publication number: WO 2015/037624

(57) **Abstract**

A method for measuring influenza A virus by an immunoassay using an anti-influenza A virus monoclonal antibody which is highly reactive with a wide range of subtypes is disclosed. The method for measuring influenza A virus includes measuring influenza A virus by an immunoassay utilizing antigen-antibody reaction between a monoclonal antibody which specifically reacts with matrix protein (M1) of influenza A virus, or an antigen-binding fragment thereof, and influenza A virus in a sample.

SEQUENCE LISTING

<110>  DENKA SEIKEN CO., LTD.

<120>  Method of measuring influenza A virus

<130>  PF000522-PCT

<160>  1

<170>  PatentIn version 3.5

<210>  1
<211>  252
<212>  PRT
<213>  Influenza A virus

<400>  1

Met Ser Leu Leu Thr Glu Val Glu Thr Tyr Val Leu Ser Ile Val Pro
1               5                   10                  15

Ser Gly Pro Leu Lys Ala Glu Ile Ala Gln Arg Leu Glu Asp Val Phe
            20                  25                  30

Ala Gly Lys Asn Thr Asp Leu Glu Ala Leu Met Glu Trp Leu Lys Thr
        35                  40                  45

Arg Pro Ile Leu Ser Pro Leu Thr Lys Gly Ile Leu Gly Phe Val Phe
    50                  55                  60

Thr Leu Thr Val Pro Ser Glu Arg Gly Leu Gln Arg Arg Arg Phe Val
65                  70                  75                  80

Gln Asn Ala Leu Asn Gly Asn Gly Asp Pro Asn Asn Met Asp Arg Ala
                85                  90                  95

Val Lys Leu Tyr Arg Lys Leu Lys Arg Glu Ile Thr Phe His Gly Ala
            100                 105                 110

Lys Glu Ile Ala Leu Ser Tyr Ser Ala Gly Ala Leu Ala Ser Cys Met
        115                 120                 125

Gly Leu Ile Tyr Asn Arg Met Gly Ala Val Thr Thr Glu Ser Ala Phe
    130                 135                 140

Gly Leu Ile Cys Ala Thr Cys Glu Gln Ile Ala Asp Ser Gln His Lys
145                 150                 155                 160

Ser His Arg Gln Met Val Thr Thr Thr Asn Pro Leu Ile Arg His Glu
                165                 170                 175

Asn Arg Met Val Leu Ala Ser Thr Thr Ala Lys Ala Met Glu Gln Met
            180                 185                 190

Ala Gly Ser Ser Glu Gln Ala Ala Glu Ala Met Glu Val Ala Ser Gln
        195                 200                 205

Ala Arg Gln Met Val Gln Ala Met Arg Ala Ile Gly Thr His Pro Ser
    210                 215                 220

Ser Ser Thr Gly Leu Lys Asn Asp Leu Leu Glu Asn Leu Gln Ala Tyr
225                 230                 235                 240

Gln Lys Arg Met Gly Val Gln Met Gln Arg Phe Lys
                245                 250

## Description

### TECHNICAL FIELD

The present invention relates to a method for measuring influenza A virus, and an immunoassay device and a monoclonal antibody to be used therefor.

### BACKGROUND ART

Influenza is an infectious disease which is prevalent during winter. Although patients with influenza develop clinical symptoms such as high fever, upper respiratory inflammation, malaise, and the like, differential diagnosis of influenza from infectious diseases caused by other viruses such as adeno virus, RS virus, parainfluenza virus, human metapneumovirus, and the like based on these symptoms alone is not always easy.

Examples of methods for definitive diagnosis of influenza include virus isolation, serological diagnosis, and nucleic acid detection (PCR). Since these methods cannot be easily and regularly carried out at clinical sites such as consultation rooms, a simpler diagnostic method has been demanded. Recently, an antigen detection reagent based on immunochromatography was developed, and has been widely used for aiding the diagnosis since the reagent enables simple and rapid diagnosis.

Influenza virus is a virus having segmental negative-strand RNA as the genomic gene, which is composed of eight segments, that is, HA, NA, PA, Pub1, PB2, M, NP, and NS segments. Influenza virus can be divided into type A, type B, and type C depending on the antigenicities of matrix protein (M1) and nucleoprotein (NP), among the proteins constituting the virus. Since the proteins have different antigenicities among the types, cross-reaction does not occur between different types.

Two different genes, M1 and M2, are encoded in the M segment of influenza A virus. A constituent protein is synthesized from each of the genes. These proteins correspond to M1 and BM2 of influenza B virus, but M2 of type A has a structure largely different from the structure of BM2 of type B. M1 is localized such that the inside of the viral envelope is lined therewith, and it is thought that M1 substantially plays a role as a shell.

As therapeutic agents for influenza, oseltamivir phosphate, zanamivir hydrate, peramivir hydrate, laninamivir octanoate hydrate, and amantadine hydrochloride are used. Since amantadine hydrochloride, which is an M2 inhibitor, does not inhibit infection and growth of influenza B virus, it is used for treatment of only influenza A virus. Although the former four therapeutic agents have the same site of action as neuraminidase (NA) inhibitors, they have been pointed out to show different effectiveness among the different types of influenza in terms of the time required for reduction of fever, the virus survival rate, and the like (Non-patent Document 2). Thus, differential diagnosis of the type of influenza is important for appropriate selection of the therapeutic agent to be used thereafter.

Influenza A virus is further divided into a plurality of subtypes depending on the antigenicities of hemagglutinin (HA) and neuraminidase (NA). Sixteen kinds of HA and nine kinds of NA have been reported to date, and restriction of the host range occurs depending on their combination. Other than the four kinds of subtypes H1N1, H3N2, H1N2, and H2N2, which are known as causes of human influenza, infection with H5N1, H7N9, and the like from other animal hosts may also rarely occur in human. Although conventional immunoassay devices show reaction with various subtypes, their reactivity is not uniform, and low in cases of some subtypes.

In conventional detection of influenza, an anti-NP antibody (Patent Documents 1 to 3), anti-M2 antibody (Patent Document 4), or the like has been particularly used. Although there are reports in which, for example, an anti-M1 antibody which undergoes antigen-antibody reaction with M1 was used in a study (Non-patent Documents 3 and 4), the anti-M1 antibody was not used for an immunoassay device for the purpose of aiding diagnosis.

### PRIOR ART DOCUMENTS

### [Patent Documents]

[Patent Document 1] JP 2007-285749 A
[Patent Document 2] JP 4936428 B
[Patent Document 3] WO 2005/007698
[Patent Document 4] JP 4932940 B

### [Non-patent Documents]

[Non-patent Document 1] Virus vol. 56(1), pp. 109-116, 2006
[Non-patent Document 2] J Infect. 2008 Jan; 56(1): 51-7. Epub 2007 Oct 15. (A comparison of the effectiveness of zanamivir and oseltamivir for the treatment of influenza A and B.)
[Non-patent Document 3] Journal of Immunological Methods Volume 387, Issues 1-2, 31 January 2013, Pages 43-50 (Preparation of monoclonal antibodies against poor immunogenic avian influenza virus proteins)
[Non-patent Document 4] Virus Genes. 1989 Nov; 3(2): 111-26. (Monoclonal antibody analysis of influenza virus matrix protein epitopes involved in transcription inhibition.)

### SUMMARY OF THE INVENTION

### PROBLEMS TO BE SOLVED BY THE INVENTION

In conventional immunoassay methods, an anti-NP monoclonal antibody or the like has been used. However, the reactivity was low in these methods depending on the subtype of the influenza A virus.

An object of the present invention is to provide a method for measuring influenza A virus by an immunoassay using an anti-influenza A monoclonal antibody which is highly reactive with a wide range of subtypes. Another object of the present invention is to provide an immunoassay device and a monoclonal antibody to be used in the method of the present invention described above.

### MEANS FOR SOLVING THE PROBLEMS

As a result of intensive study, the present inventors succeeded in preparation of an anti-influenza A monoclonal antibody which specifically reacts with influenza A virus, using M1 of influenza A virus as an antigen, and discovered that use of this monoclonal antibody in an immunoassay of influenza A virus enables measurement of influenza A virus subtypes which have been difficult to detect by conventional immunoassays using an anti-NP monoclonal antibody, thereby completing the present invention.

That is, the present invention provides a method for measuring influenza A virus, which method comprises measuring influenza A virus by an immunoassay utilizing antigen-antibody reaction between a monoclonal antibody which specifically reacts with matrix protein (M1) of influenza A virus, or an antigen-binding fragment thereof, and influenza A virus in a sample.

The present invention also provides an immunoassay device comprising a detection area in which a first antibody or an antigen-binding fragment thereof is immobilized on a support, a label area in which a second antibody or an antigen-binding fragment thereof is supplied together with a sample, and a sample movement area, wherein at least one of the first antibody and the second antibody is a monoclonal antibody which specifically reacts with matrix protein (M1) of influenza A virus. The present invention also provides a monoclonal antibody which specifically reacts with matrix protein (M1) of influenza A virus, or an antigen-binding fragment thereof.

### EFFECT OF THE INVENTION

By the method of the present invention, an immunoassay for a wide range of subtypes of influenza A virus is possible. The present invention provides a novel immunoassay device and a novel monoclonal antibody to be used for the method of the present invention described above.

### BRIEF DESCRIPTION OF THE DRAWINGS

Fig. 1 is a diagram schematically illustrating a preferred embodiment of the immunochromatographic immunoassay device of the present invention.
Fig. 2 is a diagram showing results of Western blot analysis on the reactivity of the monoclonal antibody of the present invention prepared in the Examples below.
Fig. 3 is a diagram showing results of a peptide array analysis for investigation of the reactivity of the monoclonal antibody of the present invention prepared in the Examples below.
Fig. 4 is a diagram showing the amino acid sequence in the C-terminal side of M1 of each subtype of influenza A virus, wherein the regions to which the monoclonal antibody of the present invention prepared in the Examples below is bound are shown.

### MODE FOR CARRYING OUT THE INVENTION

The monoclonal antibody of the present invention undergoes antigen-antibody reaction with influenza A virus M1 (hereinafter referred to as A-M1). A-M1 is a protein constituted by 252 amino acid residues, and its signal due to antigen-antibody reaction can be specifically detected at a molecular weight of 20 to 35 kD when the antibody is used for detection by Western blotting. The term "specific" in the present description means that, in a liquid system containing a mixture of proteins and the antibody, the antibody does not cause antigen-antibody reaction with the proteins other than A-M1 at a detectable level, or, even in cases where the antibody causes a certain binding reaction or association reaction with a protein other than A-M1, the reaction is evidently weaker than the antigen-antibody reaction between the antibody and A-M1.

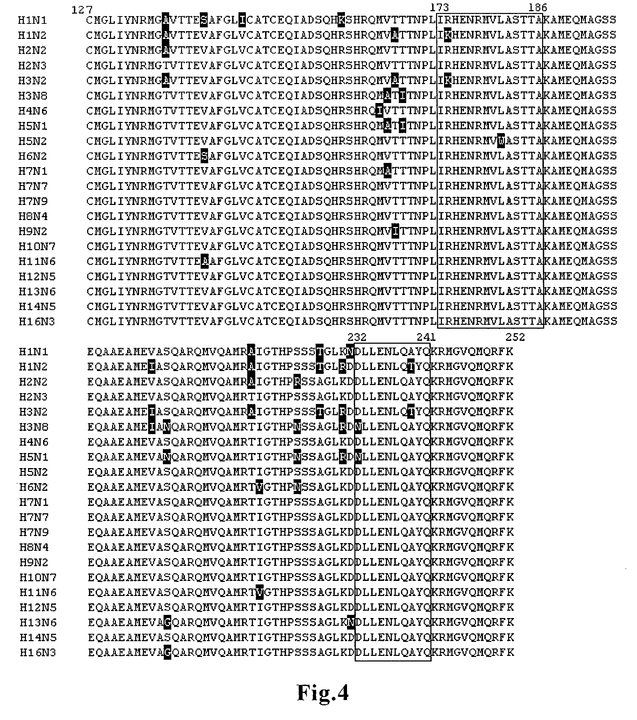

In a preferred mode, the monoclonal antibody of the present invention reacts with the region of the 127th to 252nd amino acids in an A-M1 amino acid sequence. Amino acid sequences of A-M1 are known, and described in, for example, GenBank: ACD37490 (SEQ ID NO:1). Fig. 4 shows comparison of sequences of the 127th to 252nd amino acids among A-M1 amino acid sequences of different subtypes.

More preferably, the monoclonal antibody of the present invention binds to the region of the 173rd to 186th amino acids or the region of the 232nd to 241 st amino acids in an A-M1 amino acid sequence (see the Examples below).

An antigen-binding site alone separated from the monoclonal antibody of the present invention may be used for the reaction. That is, fragments having specific antigen-binding capacity (antigen-binding fragment), such as Fab, Fab', F(ab')₂, and single-chain antibodies (scFv) prepared by known methods are included within the scope of the present invention. The class of the monoclonal antibody is not limited to IgG, and may also be IgM or IgY.

The monoclonal antibody of the present invention specifically reacts with M1 of the subtypes H1N1, H1N2, H2N2, H2N3, H3N2, H3N8, H4N6, H5N1, H5N2, H6N2, H7N1, H7N7, H7N9, H8N4, H9N2, H10N7, H11N6, H12N5, H13N6, H14N5, H15N8, and H16N3 of influenza A virus. More preferably, the monoclonal antibody of the present invention specifically reacts with M1 of any known strain of influenza A virus (see Examples below).

In a preferred embodiment, the monoclonal antibody used in the present invention does not undergo antigen-antibody reaction with pathogens of other infectious diseases. For example, the monoclonal antibody does not undergo antigen-antibody reaction with influenza B virus, adeno virus, Coxsackie virus, echovirus, herpes simplex virus, measles virus, mumps virus, parainfluenza virus, RS virus, *Chlamydia psittaci, Chlamydia trachomatis, Mycoplasma pneumoniae, Bordetella pertussis, Escherichia coli, Haemophilus influenzae, Legionella pneumophila, Listeria monocytogenes, Pseudomonas aeruginosa, Serratia marcescens, Staphylococcus aureus, Staphylococcus epidermidis, Streptococcus pneumoniae, Streptococcus pyogenes,* group B streptococci, group C streptococci, or group G streptococci.

The monoclonal antibody used in the present invention may be obtained by immunizing an animal with A-M1 or a partial peptide thereof (the polypeptide composed of the region of the 127th to 252nd amino acids, or a partial peptide thereof) by a known immunological method, and then preparing a hybridoma using cells of the immunized animal. The length of the peptide used for the immunization is not limited. Preferably, a peptide of not less than 5 amino acids, more preferably not less than 10 amino acids, may be used to provide the immunogen.

The A-M1 to be used as the immunogen can be obtained from a cultured virus liquid, or can be obtained by incorporating DNA encoding A-M1 into a plasmid vector, and introducing the resulting vector to a host cell, followed by allowing expression of the A-M1.

Alternatively, the A-M1 or the partial peptide thereof to be used as the immunogen can be expressed as a fusion protein with a protein exemplified below, and the expressed fusion protein can be used as the immunogen after purification or without purification. The preparation of the fusion protein can be carried out using, for example, Glutathion S-transferase (GST), maltose-binding protein (MBP), thioredoxin (TRX), Nus-tag, S-tag, HSV-tag, FRAG tag, polyhistidine tag, or the like which is commonly used as a "protein expression/purification tag" by those skilled in the art. Preferably, the fusion protein with such a protein is cleaved into the portion of M1 or the partial peptide thereof, and the tag portion, using a digestive enzyme, and subjected to separation/purification before use as the immunogen.

The preparation of the monoclonal antibody from the immunized animal can be easily carried out by the well-known method of Kohler et al. (Kohler et al., Nature, vol. 256, p. 495-497 (1975)). That is, antibody-producing cells such as spleen cells or lymphocytes are recovered from the immunized animal, and the recovered cells are fused with mouse myeloma cells by a conventional method to prepare hybridomas. The resulting hybridomas are cloned by the limiting dilution method or the like. Thereafter, a monoclonal antibody that undergoes antigen-antibody reaction with A-M1 is selected from the monoclonal antibodies produced by the cloned hybridomas.

Purification of the monoclonal antibody from ascites or a culture supernatant may be carried out by a known immunoglobulin purification method. Examples of the method include fractionation by salting out using ammonium sulfate or sodium sulfate, PEG fractionation, ethanol fractionation, DEAE ion-exchange chromatography, and gel filtration. Depending on the species of the immunized animal and the class of the monoclonal antibody, affinity chromatography using a carrier to which any of protein A, protein G, and protein L is bound may be used for the purification.

In the method for measuring influenza A virus of the present invention, influenza A virus is measured by an immunoassay utilizing antigen-antibody reaction between an anti-influenza A virus M1 antibody (which may be hereinafter referred to as "anti-A-M1 antibody") prepared as described above or an antigen-binding fragment thereof, and influenza A virus in a sample. As the method of the immunoassay, any of methods that are well known to those skilled in the art may be used. Examples of the method include competition immunoassays, agglutination immunoassays, Western blotting, immunostaining, and sandwich methods. In the present invention, "measurement" includes any of quantification, semi-quantification, and detection.

In an immunoassay based on the detection principle of the so-called sandwich method comprising a step of forming a complex containing an antibody immobilized on a solid phase (Antibody 1) (unless otherwise apparent from the context, the term "antibody" means "antibody or antigen-binding fragment thereof" in the following part of the present description before Examples), a labeled antibody (Antibody 2), and A-M1, an anti-A-M1 antibody that reacts with the region of the 127th to 252nd amino acids in the A-M1 amino acid sequence is preferably used as either one of Antibody 1 and Antibody 2. More preferably, an anti-A-M1 antibody which binds to the region of the 173rd to 186th amino acids or the region of the 232nd to 241 st amino acids in the A-M1 amino acid sequence is used as either one of Antibody 1 and Antibody 2. Still more preferably, an antibody which binds to the region of the 173rd to 186th amino acids in the A-M1 amino acid sequence is used as either one of Antibody 1 and Antibody 2, and an anti-A-M1 antibody which binds to the region of the 232nd to 241st amino acids in the A-M1 amino acid sequence is used as the other one of Antibody 1 and Antibody 2. These antibodies, of course, can bind to A-M1 at the same time.

The monoclonal antibody of the present invention binds to a wide range of subtypes of influenza A virus with high affinity. Conventionally-used antibodies against nucleoprotein (NP) of influenza A virus are also capable of binding to several subtypes with high affinity, and some of the known anti-A-NP antibodies bind to some subtypes with higher affinity compared to the monoclonal antibody of the present invention. Thus, by using a mixture of the monoclonal antibody of the present invention and a known anti-A-NP antibody as the antibody for the immunoassay, the measurement sensitivity for a wide range of subtypes can be further increased (see Example 5 below).

The immunoassay is preferably carried out by a sandwich method. The sandwich method *per se* is well known in the field of immunoassays, and can be carried out by, for example, immunochromatography or ELISA. These sandwich methods *per se* are well known, and the method of the present invention can be carried out in the same manner as a well-known sandwich method except that the anti-A-M1 antibody of the present invention described above is used.

In the immunoassay based on the detection principle of a sandwich method, any solid phase may be used as the solid phase on which the antibody is to be immobilized, as long as the antibody can be immobilized thereon by a known technique. The solid phase may be arbitrarily selected from known solid phases such as porous thin films (membranes) having a capillary action; particles, test tubes, and resin plates. Examples of the substance for labeling the antibody include enzymes, radioisotopes, fluorescent substances, luminescent substances, colored particles, and colloidal particles.

Among the above-mentioned immunoassay methods using various materials, lateral-flow immunoassay methods using a membrane are preferred particularly from the viewpoint of enabling simple and rapid clinical tests.

The present invention also provides an immunoassay device which enables a lateral-flow immunoassay using an anti-A-M1 antibody. The immunoassay device provided by the present invention is composed of a support having a detection area in which an antibody (Antibody 1) for capturing the subject to be measured (antigen) is immobilized; a label area having a movable labeled antibody (Antibody 2), a sample pad to which a sample is added dropwise, an absorption zone which absorbs a developed sample liquid, and a backing sheet for laminating these members together, wherein at least one of Antibody 1 and Antibody 2 is the anti-A-M1 antibody of the present invention.

The number of detection areas, and the number of types of the labeled antibody contained in the label area, are not limited to one. By using antibodies corresponding to a plurality of subjects to be measured, two or more antigens can be detected in a single immunoassay device.

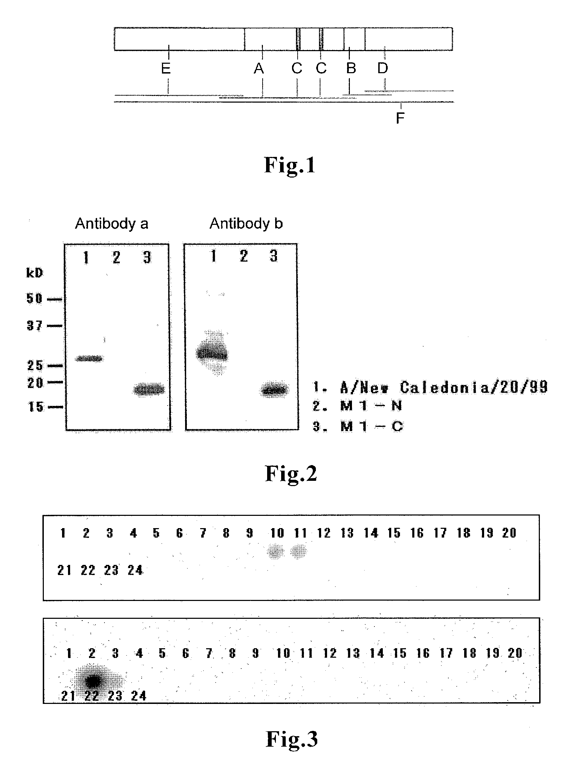

Fig. 1 is a diagram illustrating a preferred embodiment of the immunoassay device of the present invention. A represents a support; B represents a label area; C represents a detection area; D represents a sample pad; E represents an absorption zone; and F represents a backing sheet.

In Fig. 1, the upper panel shows a top view, and the lower panel shows a cross-sectional view. In the case shown in this figure, a support wherein two detection areas are formed on a backing sheet; an absorption zone; a label area; and a sample pad; are laminated together. As shown in the figure, an end of the absorption zone overlaps with an end of the support; the other end of the support overlaps with an end of the label area; and the other end of the label area overlaps with an end of the sample pad. By this, a continuous lateral-flow channel is formed.

The support is a material having a property which allows immobilization of the antibody for capturing the antigen, as well as a property which does not prevent horizontal movement of a liquid. The support is preferably a porous thin film having a capillary action, which is a material capable of transporting, by absorption, a liquid and a component dispersed in the liquid. The material constituting the support is not limited, and examples of the material include cellulose, nitrocellulose, cellulose acetate, polyvinylidene difluoride (PVDF), glass fiber, nylon, and polyketone. Among these, a thin film prepared using nitrocellulose is more preferred.

The label area is composed of a porous base material containing a labeled antibody. The material of the base material may be a glass fiber, non-woven fabric, or the like which is commonly used. The base material is preferably pad-shaped and has a thickness of about 0.3 mm to 0.6 mm from the viewpoint of allowing impregnation with a large amount of labeled antibody.

The detection area means a partial area(s) of the support on which the antibody for capturing the antigen is immobilized. From the viewpoint of practically aiding diagnosis, the detection area preferably has at least one area where the anti-A-M1 antibody for capturing the A-M1 antigen is immobilized, as well as a detection area for detection of influenza B virus.

The sample pad is a portion to which a sample, or a specimen prepared using a sample, is added dropwise. The sample pad is a water-absorptive, porous material. The material of the sample pad may be cellulose, glass fiber, non-woven fabric, or the like which is commonly used. For use of a large amount of sample in the immunoassay, the sample pad preferably has a pad shape having a thickness of about 0.3 mm to 1 mm. It should be noted that the distinction between the sample pad and the label area is merely based on their functions, and they do not necessarily need to be separate materials. That is, a partial area in the material placed as the sample pad may have a function as the label area.

The absorption zone is a member for absorbing components that are supplied to the support but not involved in the reaction in the detection area. The material of the absorption zone may be a filter paper, sponge, or the like having high water holding capacity composed of a common natural macromolecular compound, synthetic macromolecular compound, or the like. For promotion of development of the sample, the absorption zone preferably has high water-absorbing capacity.

The backing sheet is a member on which all of the above-described materials, that is, the support, sample pad, label area, and absorption zone, are attached/immobilized such that they partially overlap with each other. The backing sheet is not necessarily required as long as these materials are arranged/immobilized at optimal intervals, but it is generally preferred to use the backing sheet from the viewpoint of convenience in production and/or use of the device.

In the immunoassay device of the embodiment illustrated in Fig. 1, the sample passes through a porous channel formed by a series of the members, that is, the sample pad, label area, support, detection area, and absorption zone, which are connected to each other. Therefore, in the present embodiment, all of these members constitute the sample movement area. Depending on the material and the shape of each constituting material, the sample may pass through an interface without impregnation into the material. Since the sample movement area defined in the present description may be located either inside the material or on an interface of the material, the immunoassay device of this embodiment is also included within the scope of the present description.

A method for using the immunoassay device of the present invention is described below based on the embodiment shown in Fig. 1.

The measurement is begun by adding a sample, or a specimen prepared using a sample, dropwise to the sample pad. The test sample to be added dropwise is preferably about 2- to 20-fold diluted in advance using a buffer containing a surfactant.

The test sample added dropwise to the sample pad is developed in the horizontal direction by the capillary action, sequentially through the label area, support, and absorption zone. As the development of the test sample proceeds, a labeled antibody is released into the liquid in the label area, and developed into the support. In cases where an antigen is present in the test sample, the antigen is specifically captured by a capture antibody in the detection area of the support, and, in addition, the antigen also forms a complex with the labeled antibody by specific reaction. By this, sandwiching of the antigen between the antibodies is achieved in the detection area, and the labeled antibody-antigen complex can be detected in the detection area.

### EXAMPLES

The present invention is described below by way of Examples. However, the present invention is not limited to the following Examples.

### (Example 1) Preparation of Anti-influenza A Virus M1 Monoclonal Antibody 1. Preparation of Influenza A Virus M1 Antigen

Based on a cDNA library constructed from viral RNA of the A/Brisbane/59/2007 strain by reverse transcription, DNA encoding M1 was subcloned into a plasmid vector. An *E. coli* strain in which the plasmid vector was introduced was subjected to aeration/agitation culture to allow expression of the M1. The M1 was recovered by disruption of the bacterial cells and subsequent centrifugation. The recovered M1 was purified by chromatography. In the present description, the purified product is referred to as "purified M1 antigen".

### 2. Preparation of Anti-influenza A Virus M1 Monoclonal Antibody

BALB/c mice were immunized with the purified M1 antigen, and kept for a certain period. From each mouse, the spleen was removed, and fusion with mouse myeloma cells (P3×63) was carried out by the method of Kohler et al. (Kohler et al., Nature, vol. 256, p. 495-497 (1975)). The resulting fused cells (hybridomas) were kept in an incubator at 37°C. The cells were then purified (into monoclonal cells) while the antibody activity in the supernatant was checked by ELISA using a plate on which the purified M1 antigen is immobilized. The obtained cell line was intraperitoneally administered to pristane-treated BALB/c mice. About two weeks later, antibody-containing ascites was collected.

From the ascites obtained, IgG was purified by affinity chromatography using a protein A column, to obtain a purified anti-influenza A virus M1 antibody.

### (Reference Example 1)

### 1. Preparation of Anti-influenza A Virus NP Monoclonal Antibody

BALB/c mice were immunized with an influenza A virus antigen, and kept for a certain period. From each mouse, the spleen was removed, and fusion with mouse myeloma cells (P3×63) was carried out by the method of Kohler et al. (Kohler et al., Nature, vol. 256, p. 495-497 (1975)). The resulting fused cells (hybridomas) were kept in an incubator at 37°C. The cells were then purified (into monoclonal cells) while the antibody activity in the supernatant was checked by ELISA using a plate on which an influenza A virus NP antigen is immobilized. The obtained cell line was intraperitoneally administered to pristane-treated BALB/c mice. About two weeks later, antibody-containing ascites was collected.

From the ascites obtained, IgG was purified by affinity chromatography using a protein A column, to obtain a purified anti-influenza A virus NP antibody. In the present description, the purified antibody is referred to as "anti-A-NP antibody".

### (Example 2) Influenza Virus Antigen Detection Reagent Using Colored Polystyrene Particles

### 1. Immobilization of Anti-influenza A Virus M1 Antibody on Nitrocellulose Membrane

The anti-A-M1 antibody prepared in Example 1 was diluted to 1.0 mg/mL with purified water. The resulting dilution was linearly applied to a predetermined position of a nitrocellulose membrane lined with a PET film. The membrane was then dried at 45°C for 30 minutes to obtain a membrane on which the anti-influenza A virus M1 antibody is immobilized. In the present description, the membrane obtained is referred to as "anti-M1 antibody-immobilized membrane".

### 2. Immobilization of Anti-influenza A Virus NP Antibody on Nitrocellulose Membrane

Using the anti-NP antibody prepared in Reference Example 1, a membrane on which the anti-influenza A virus NP antibody is immobilized was obtained by the same method as in Example 2-1. In the present description, the membrane obtained is referred to as "anti-NP antibody-immobilized membrane".

### 3. Immobilization of Anti-influenza A Virus M1 Antibody and Anti-influenza A Virus NP Antibody on Nitrocellulose Membrane

The anti-A-M1 antibody prepared in Example 1 and the anti-A-NP antibody prepared in Reference Example 1 were mixed together at a predetermined ratio, and the resulting mixture was diluted to 1.0 mg/mL with purified water. The resulting dilution was linearly applied to a predetermined position of a nitrocellulose membrane lined with a PET film. The membrane was then dried at 45°C for 30 minutes to obtain a membrane on which the anti-influenza A virus M1 antibody + anti-influenza A virus NP antibody are immobilized. In the present description, the membrane obtained is referred to as "anti-M1+NP antibodies-immobilized membrane".

### 4. Immobilization of Anti-influenza A Virus M1 Antibody on Colored Polystyrene Particles

The anti-A-M1 antibody prepared in Example 1 was diluted to 1.0 mg/mL with purified water. To the resulting dilution, colored polystyrene particles were added at 0.1%, and the resulting mixture was stirred. Carbodiimide was then added to the mixture at 1%, and the resulting mixture was stirred. After removing the supernatant by centrifugation, the precipitate was resuspended in 50 mM Tris (pH 9.0) supplemented with 3% BSA, to obtain colored polystyrene particles to which the anti-influenza A virus M1 antibody is bound. In the present description, the particles obtained are referred to as "anti-M1 antibody-immobilized particles".

### 5. Immobilization of Anti-influenza A Virus NP Antibody on Colored Polystyrene Particles

Using the anti-A-NP antibody prepared in Reference Example 1, colored polystyrene particles to which the anti-influenza A virus NP antibody is bound were obtained by the same method as in Example 2-3. In the present description, the particles obtained are referred to as "anti-NP antibody-immobilized particles".

### 6. Immobilization of Anti-influenza A Virus M1 Antibody and Anti-influenza A Virus NP Antibody on Colored Polystyrene Particles

The anti-A-M1 antibody prepared in Example 1 and the anti-A-NP antibody prepared in Reference Example 1 were mixed together at a predetermined ratio, and the resulting mixture was diluted to 1.0 mg/mL with purified water. To the resulting dilution, colored polystyrene particles were added at 0.1%, and the resulting mixture was stirred. Carbodiimide was then added to the mixture at 1%, and the resulting mixture was stirred. After removing the supernatant by centrifugation, the precipitate was resuspended in 50 mM Tris (pH 9.0) supplemented with 3% BSA, to obtain colored polystyrene particles to which the anti-influenza A virus M1 antibody + anti-influenza A virus NP antibody are bound. In the present description, the particles obtained are referred to as "anti-M1+NP antibodies-immobilized particles".

### 7. Application/Drying of Colored Polystyrene Particles to Which Anti-influenza A Virus Antibody is Bound

A predetermined amount, 1.0 µg, of the antibody-immobilized particles prepared in 4 or 5 were applied to a glass-fiber non-woven fabric, and the non-woven fabric was then dried at 45°C for 30 minutes. In the present description, the non-woven fabric obtained is referred to as "dry pad".

In addition, separately from the above process, a predetermined amount, 1.0 µg, of the anti-M1+NP antibodies-immobilized particles prepared in 6 were applied to a glass-fiber non-woven fabric, and the non-woven fabric was then dried at 45°C for 30 minutes. In the present description, the non-woven fabric obtained is referred to as "M1+NP dry pad".

### 8. Preparation of Influenza A Virus Test Pieces

The antibody-immobilized membrane prepared in 1 or 2, and the dry pad prepared in 7, were laminated with other members (backing sheet, absorption zone, and sample pad), and the resulting laminate was cut into a piece with a width of 5 mm, to provide an influenza A virus test piece. In the present description, the test piece is referred to as "M1 test piece". In the preparation of the M1 test piece, the combination of the antibody-immobilized membrane and the dry pad to be laminated was selected such that at least one of these contains the anti-A-M1 antibody.

In addition, separately from the above process, the anti-M1+NP antibodies-immobilized membrane prepared in 3 and the M1+NP dry pad prepared in 7 were laminated with other members (backing sheet, absorption zone, and sample pad), and the resulting laminate was cut into a piece with a width of 5 mm, to provide an influenza A virus M1+NP test piece. In the present description, the test piece is referred to as "M1+NP test piece".

### 9. Detection of Influenza A Virus Antigen

To the sample pad of the M1 test piece or the M1+NP test piece prepared in 8, 60 µL of a sample suspension containing an influenza A virus antigen (10 mM ADA (pH 6.0), 2% polyoxyethylene cetyl ether, 1% polyoxyethylene octylphenyl ether, 0.25 M potassium chloride, 0.25 M lithium chloride, and 3% BSA) was added dropwise, and the resulting mixture was left to stand for 8 minutes. In cases where antibody-immobilized particles which had reacted with the antigen were captured at the position where the antibody was applied on the antibody-immobilized membrane, and coloring due to the capture could be visually observed, the test piece was evaluated as "+". In cases where no coloring could be visually observed, the test piece was evaluated as "-". In this test, the test pieces containing any of the combinations, that is, the test pieces containing a combination in which one or both of the antibody-immobilized membrane and the dry pad contain(s) the anti-A-M1 antibody, were evaluated as "+".

### (Example 3) Analysis of Antigen-recognizing Regions (Epitopes) of Anti-A-M1 Antibody

### 1. Preparation of Influenza A Virus Recombinant M1

Based on the cDNA library constructed in Example 1-1, each of DNA encoding the 1st to 126th amino acids and DNA encoding the 127th to 252nd amino acids in an M1 amino acid sequence was subcloned into a plasmid vector. An E. *coli* strain in which the plasmid vector was introduced was subjected to aeration/agitation culture to allow expression of the recombinant M1. Each recombinant M1 was recovered by disruption of the bacterial cells and subsequent centrifugation. The recovered recombinant M1 was purified by chromatography. In the present description, the recombinant M1 composed of the former amino acid sequence is referred to as "M1-N", and the recombinant M1 composed of the latter amino acid sequence is referred to as "M1-C".

### 2. Confirmation of Reactivity by Western Blotting

Reactivity of the antibody obtained in Example 1 was confirmed using the A/New Caledonia/20/99 strain, M1-N, and M1-C. SDS-PAGE was carried out by a conventional method using 10% acrylamide gel. Thereafter, protein was transferred to a PVDF membrane. After blocking of the membrane using skim milk, the membrane was sufficiently washed with PBS-Tween. The membrane was then allowed to react with the anti-A-M1 antibody whose concentration was adjusted to 1 µg/mL using PBS-Tween, at room temperature for 1 hour. After sufficiently washing the membrane with PBS-Tween, the membrane was allowed to react with a 3000-fold diluted HRP-labeled anti-mouse antibody at room temperature for 1 hour. After sufficiently washing the membrane with PBS-Tween, signals were detected using a chemiluminescence detection reagent. The antibody tested could be confirmed to be reactive with the A/New Caledonia/20/99 strain. The antibody was not reactive with M1-N, but was reactive with M1-C. The results are shown in Fig. 2.

### 3. Analysis of Epitope Using Peptide Array

For further clarification of the epitope, partial peptides of M1-C were used. Each of the peptides was composed of a sequence of 10 or 11 consecutive amino acids in the M1-C sequence. The peptides were immobilized at regular intervals on a cellulose membrane. The sequences of the peptides, and the numbers indicating the positions where the peptides were immobilized, are listed in Table 1. The membrane was blocked with skim milk, and sufficiently washed with TBS-Tween. The membrane was then allowed to react with the anti-A-M1 antibody whose concentration was adjusted to 1 µg/mL using TBS-Tween, at room temperature for 1 hour. After sufficiently washing the membrane with TBS-Tween, the membrane was allowed to react with a 3000-fold diluted HRP-labeled anti-mouse antibody at room temperature for 1 hour. The membrane was washed with TBS-Tween, and then sufficiently with TBS. Thereafter, signals were detected using a chemiluminescence detection reagent. The results are shown in Fig. 3. The antibody a was reactive with the peptides at the positions 10 and 11, and the antibody b was reactive with the peptide at the position 22. The amino acid sequences that were assumed to be epitopes of the antibody a and the antibody b are shown in Fig. 4.

**[Table 1]**

| Position | Sequence |
|---|---|
| 1 | CMGLIYNRMG |
| 2 | YNRMGAVTTE |
| 3 | AVTTESAFGL |
| 4 | SAFGLICATC |
| 5 | ICATCEQIAD |
| 6 | EQIADSQHKS |
| 7 | SQHKSHRQMV |
| 8 | HRQMVTTTNP |
| 9 | TTTNPLIRHE |
| 10 | LIRHENRMVL |
| 11 | NRMVLASTTA |
| 12 | ASTTAKAMEQ |
| 13 | KAMEQMAGSS |
| 14 | MAGSSEQAAE |
| 15 | EQAAEAMEVA |
| 16 | AMEVASQARQ |
| 17 | SQARQMVQAM |
| 18 | MVQAMRAIGT |
| 19 | RAIGTHPSSS |
| 20 | HPSSSTGLKN |
| 21 | TGLKNDLLEN |
| 22 | DLLENLQAYQ |
| 23 | LQAYQKRMGV |
| 24 | KRMGVQMQRFK |

### (Example 4) Immunoassay of Strains of Influenza A Virus Subtypes Using Immunoassay Devices

### 1. Preparation of Immunoassay Device Comprising Anti-A-M1 Antibody

A test piece was prepared using the antibody a and the antibody b of Example 3-3 for the antibody-immobilized membrane and the dry pad, by the same method as described in Example 2 (device 1 of the present invention).

### 2. Preparation of Immunoassay Device Comprising Anti-NP Antibody

As a control, a test piece was prepared using the anti-NP-immobilized membrane and the anti-NP-immobilized particles by the same method as described in Example 2-6 (conventional device).

### 3. Immunoassay of Strains of Influenza A Virus Subtypes

To the sample pad of the immunoassay device prepared in 1, 50 µL of a sample suspension containing an influenza A virus antigen was added dropwise, and the resulting mixture was left to stand for 8 minutes. In cases where antibody-immobilized particles which had reacted with the antigen were captured at the position where the antibody was applied on the antibody-immobilized membrane, and coloring due to the capture could be visually observed, the device was evaluated as "+". In cases where no coloring could be visually observed, the device was evaluated as "-". The monoclonal antibody of the present invention was evaluated as "+" in the assays of the subtypes of influenza A virus of H1N1, H2N2, H2N3, H3N2, H3N8, H4N6, H5N1, H5N2, H6N2, H7N1, H7N7, H7N9, H8N4, H9N2, H10N7, H11N6, H12N5, H13N6, H14N5, H15N8, and H16N3.

### 4. Comparison of Reactivity with Strains of Influenza A Virus Subtypes

Using the immunoassay devices prepared in 1 and 2, immunoassays were carried out by the same method as in 3, and the reactivity was compared between the device 1 of the present invention and the conventional device. While the conventional device failed to detect seven subtypes (12 virus strains), all of these seven subtypes could be detected with the device 1 of the present invention. Thus, the device 1 of the present invention was shown to have higher detection sensitivity than the conventional device. The results are shown in Table 2.

**[Table 2]**

| Subtype | Strain name | Conventional device | Device of present invention |
|---|---|---|---|
| H1N1 | A/duck/Tottori/723/80 | + | + |
| H1N1 | A/Hokkaido/11/2002 | - | + |
| H1N1pdm | A/Hyogo/YS/2011 | - | + |
| H2N2 | A/murakami/4/64 | + | + |
| H2N3 | A/duck/Hokkaido/17/01 | + | + |
| H3N2 | A/Hokkaido/30-1-a/2013 | + | + |
| H3N8 | A/duck/Mongolia/4/03 | + | + |
| H4N6 | A/duck/Czech/56 | + | + |
| H5N1 | A/Hong Kong/483/1997 * | - | + |
| H5N1 | A/Vietnam/1194/04 * | + | + |
| H5N1 | A/whooper swan/Mongolia/3/2005 * | - | + |
| H5N1 | A/whooper swan/Hokkaido/1/2008 * | - | + |
| H5N1 | A/whooper swan/Hokkaido/4/2011 * | + | + |
| H5N1 | A/duck/Vietnam/OIE-559/2011 * | - | + |
| H5N2 | A/duck/Pennsylvania/10218/84 | + | + |
| H6N2 | A/turkey/Massachusetts/3740/65 | + | + |
| H7N1 | A/turkey/Italy/4580/1999 * | + | + |
| H7N7 | A/seal/Massachusetts/1/80 | - | + |
| H7N7 | A/chicken/Netherlands/2586/2003 * | + | + |
| H7N9 | A/duck/Mongolia/119/2008 | + | + |
| H7N9 | A/duck/Mongolia/128/2008 | - | + |
| H7N9 | A/duck/Mongolia/147/2008 | - | + |
| H7N9 | A/duck/Mongolia/129/2010 | + | + |
| H7N9 | A/Anhui/1/2013 | + | + |
| H8N4 | A/turkey/Ontario/68 | - | + |
| H9N2 | A/turkey/Wisconsin/66 | + | + |
| H10N7 | A/chicken/Germany/N/49 | + | + |
| H11N6 | A/duck/England/1/56 | + | + |
| H12N5 | A/duck/Alberta/60/76 | + | + |
| H13N6 | A/gull/Maryland/704/77 | + | + |
| H14N5 | A/mallard/Astrakhan/263/82 | + | + |
| H15N8 | A/duck/Australia/341 /83 | - | + |
| H16N3 | A/black-headed gull/Sweden/5/99 | - | + |

### (Example 5) Comparison of Detection Sensitivity for Influenza A Virus among Influenza A Virus M1 Test Piece, Influenza A Virus NP Test Piece, and Influenza A Virus M1+NP Test Piece

### 1. Preparation of Immunoassay Device Comprising Anti-A-M1 Antibody and Anti-A-NP Antibody

The M1+NP test piece prepared in Example 2-8 was used as it is (device 2 of the present invention).

### 2. Preparation of Immunoassay Device Comprising Anti-A-M1 Antibody and Immunoassay Device Comprising Anti-A-NP Antibody

The device 1 of the present invention and the conventional device prepared in Example 4-1 and 4-2 were used as they are to provide controls.

### 3. Comparison of Detection Sensitivity for Influenza A Virus among Assay Devices

To the sample pad of each of the immunoassay devices prepared in 1 and 2, 50 µL of a sample suspension prepared by diluting influenza A virus (A/New Caledonia/20/99) as appropriate was added dropwise, and each device was left to stand for 8 minutes. In cases where antibody-immobilized particles which had reacted with the antigen were captured at the position where the antibody was applied on the antibody-immobilized membrane, and coloring due to the capture could be visually observed, the device was evaluated as "+". In cases where no coloring could be visually observed, the device was evaluated as "-". As a result, the device 1 of the present invention showed a lower detection sensitivity than the conventional device, but the device 2 of the present invention showed a performance which was almost the same as that of the conventional device. It was thus found that, depending on the strain of influenza A virus, a higher sensitivity can be achieved by detecting NP. However, it was shown that the variability of the detection sensitivity among the strains can be reduced by using the combination of the detection of M1 and the detection of NP. The results are shown in Table 3.

**[Table 3]**

| | Dilution rate of the antigen | | | | |
|---|---|---|---|---|---|
| | ×160 | ×320 | ×640 | ×1280 | ×2560 |
| Conventional device | + | + | + | + | - |
| Device 1 of the present invention | + | ± | - | - | - |
| Device 2 of the present invention | + | + | + | + | - |

### DESCRIPTION OF SYMBOLS

A. Support
B. Label area
C. Detection area
D. Sample pad
E. Absorption zone
F. Backing sheet

## Claims

1. A method for measuring influenza A virus, said method comprising measuring influenza A virus by an immunoassay utilizing antigen-antibody reaction between a monoclonal antibody which specifically reacts with matrix protein (M1) of influenza A virus, or an antigen-binding fragment thereof, and influenza A virus in a sample.

2. The method according to claim 1, wherein said monoclonal antibody undergoes antigen-antibody reaction with each of the influenza A virus subtypes H1N1, H1N2, H2N2, H2N3, H3N2, H3N8, H4N6, H5N1, H5N2, H6N2, H7N1, H7N7, H7N9, H8N4, H9N2, H10N7, H11N6, H12N5, H13N6, H14N5, H15N8, and H16N3.

3. The method according to claim 1 or 2, wherein said monoclonal antibody binds to the region of the 127th to 252nd amino acids of matrix protein (M1) of influenza A virus.

4. The method according to claim 3, wherein said monoclonal antibody binds to the region of the 173rd to 186th amino acids of matrix protein (M1) of influenza A virus.

5. The method according to claim 3, wherein said monoclonal antibody binds to the region of the 232nd to 241st amino acids of matrix protein (M1) of influenza A virus.

6. The method according to any one of claims 1 to 5, wherein said immunoassay is a sandwich method.

7. The method according to claim 6, which uses two kinds of monoclonal antibodies or antigen-binding fragments thereof which are capable of binding to matrix protein (M1) of influenza A virus at the same time.

8. The method according to claim 7, wherein both of said two kinds of monoclonal antibodies bind to the region of the 127th to 252nd amino acids of matrix protein (M1) of influenza A virus.

9. The method according to claim 8, using a first monoclonal antibody which binds to the region of the 173rd to 186th amino acids of matrix protein (M1) of influenza A virus, or an antigen-binding fragment thereof, and a second monoclonal antibody which binds to the region of the 232nd to 241st amino acids of matrix protein (M1) of influenza A virus, or an antigen-binding fragment thereof.

10. The method according to any one of claims 6 to 9, wherein said immunoassay is immunochromatography.

11. The method according to any one of claims 1 to 10, using a mixture of a monoclonal antibody which specifically reacts with matrix protein (M1) of influenza A virus, or an antigen-binding fragment thereof, and a monoclonal antibody which specifically reacts with nucleoprotein (NP) of influenza A virus, or an antigen-binding fragment thereof.

12. An immunoassay device comprising a detection area in which a first antibody or an antigen-binding fragment thereof is immobilized on a support, a label area in which a second antibody or an antigen-binding fragment thereof is supplied together with a sample, and a sample movement area, wherein at least one of said first antibody and said second antibody is a monoclonal antibody which specifically reacts with matrix protein (M1) of influenza A virus.

13. The immunoassay device according to claim 12, wherein said first and second antibodies are two kinds of monoclonal antibodies which are capable of binding to matrix protein (M1) of influenza A virus at the same time.

14. A monoclonal antibody which specifically reacts with matrix protein (M1) of influenza A virus, or an antigen-binding fragment thereof.

15. The monoclonal antibody according to claim 14, which undergoes antigen-antibody reaction with each of the influenza A virus subtypes H1N1, H1N2, H2N2, H2N3, H3N2, H3N8, H4N6, H5N1, H5N2, H6N2, H7N1, H7N7, H7N9, H8N4, H9N2, H10N7, H11N6, H12N5, H13N6, H14N5, H15N8, and H16N3, or an antigen-binding fragment thereof.

16. The monoclonal antibody according to claim 14 or 15, which binds to the region of the 127th to 252nd amino acids of matrix protein (M1) of influenza A virus, or an antigen-binding fragment thereof.

17. The monoclonal antibody according to claim 16, which binds to the region of the 173rd to 186th amino acids of matrix protein (M1) of influenza A virus, or an antigen-binding fragment thereof.

18. The monoclonal antibody according to claim 17, which binds to the region of the 232nd to 241st amino acids of matrix protein (M1) of influenza A virus, or an antigen-binding fragment thereof.
